# EUROPEAN PATENT APPLICATION

(11) **EP 4 796 150 A1**
(43) Date of publication of application: **26.08.2026**
(21) Application number: 25159693.8
(22) Date of filing: 24.02.2025
(51) Int. Cl.: A61M 15/00, A24F 40/05, A24F 40/10, A61M 15/06, A61M 11/00

(54) **HUMIDIFYING HEALTH CIGARETTE INCLUDED IN THE SPINA TECHNIQUE**

(71) Applicant: Spina, Andrew, 1500 Zejtun (MT)
(72) Inventor: Spina, Andrew, 1500 Zejtun (MT)

(57) **Abstract**

The invention provides a Humidifying Health Cigarette (HHC) that generates water vapor through ultrasonic misting technology, offering a portable and discrete solution for smoking cessation without delivering nicotine. The device works in conjunction with existing nicotine replacement therapies. The invention also includes a methodology for nicotine weaning, The Spina Technique (TST), which involves gradual nicotine dose reduction, higher doses in the morning, and PRN dosing for breakthrough cravings. This combined approach provides a flexible and personalized strategy for managing nicotine addiction while reducing dependency.

Airway humidification is potentially a health benefit helping dryness and throat irritation. Infection control measures are taken into consideration in materials use, mouthpiece cleaning and temperatures.

## Description

**Field of the Invention:** The present invention relates to a **Humidifying Health Cigarette (HHC)** designed to provide a mist of water vapor for inhalation, in combination with **The Spina Technique (TST)** for gradual nicotine weaning and stress management through PRN dosing. The HHC device facilitates the delivery of water vapor while allowing for personalized nicotine replacement strategies, which includes higher morning doses, slow nicotine weaning, and as-needed (PRN) dosing to manage cravings.

**Background of the Invention:** Nicotine addiction remains a widespread challenge, and while existing nicotine replacement therapies (NRTs) such as patches, gums, and inhalers are effective, they lack flexibility and may not address the user's psychological and stress-induced cravings effectively. Furthermore, traditional NRTs do not offer a portable, discrete method for continuous use.

This invention addresses these gaps by providing a **Humidifying Health Cigarette (HHC),** a device that produces water vapor without any nicotine, while users still receive nicotine from other replacement methods. **The Spina Technique (TST),** associated with the device, enables long-term, gradual weaning and the possibility of higher nicotine doses in the morning, along with PRN dosing for breakthrough cravings, making it a comprehensive system for managing nicotine addiction.

**Summary of the Invention:** The invention provides a **Humidifying Health Cigarette (HHC),** a device that produces water vapor through ultrasonic misting technology, for use in conjunction with nicotine replacement methods. The device does not contain nicotine but serves to mimic the sensation of smoking without the harmful effects, supporting the user through the withdrawal process.

**The Spina Technique (TST)** is a methodology for nicotine weaning that utilizes HHC for vapor inhalation, combined with existing nicotine replacement therapies. The methodology includes gradual weaning, the option for PRN nicotine dosing, and higher nicotine doses in the morning to reduce cravings during high-stress periods. This combined approach allows the user to manage their addiction in a flexible, personalized manner while reducing overall nicotine dependency.

### Start: Activate Ultrasonic Misting Device

The device is powered on, initiating the misting process.

### Use Sterile Filtered Water

Only sterilized or filtered water is used to prevent contamination.

### Ultrasonic Vibrations Generate Fine Mist

High-frequency vibrations break the water into tiny mist particles.

### Mist is Delivered via Controlled Flow

A controlled mechanism ensures the mist is delivered at an optimal rate for inhalation.

### Integrated Filtration Removes Contaminants

Built-in filtration prevents microbial contamination before mist reaches the user.

### Periodic Disinfection of Device Components

The device undergoes regular cleaning and disinfection to prevent microbial buildup.

### Safe, Hygienic Misting for Inhalation

Ensures a clean and effective delivery of mist for user inhalation.

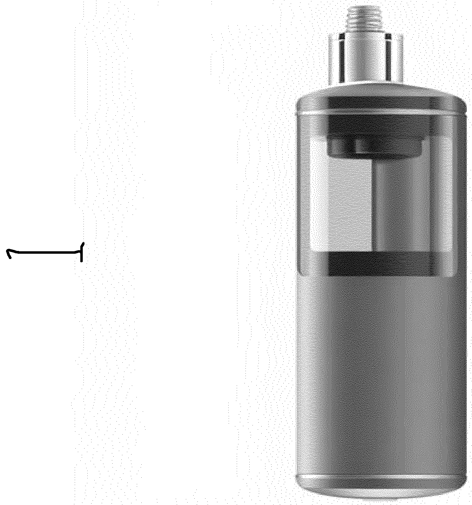

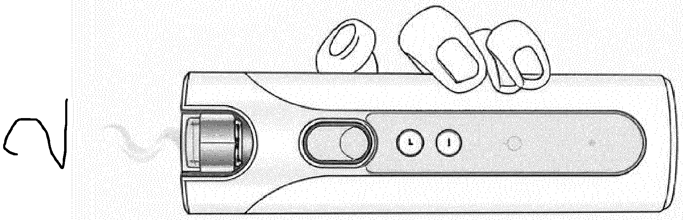

## Claims

1. A Humidifying Health Cigarette (HHC), comprising.
An ultrasonic misting mechanism that produces water vapor for inhalation.
A housing that facilitates portable, discrete use.
A control interface to adjust the misting output.

2. The Humidifying Health Cigarette (HHC) of claim 1, wherein the device is designed to operate with existing nicotine replacement therapies, not containing nicotine itself.

3. The Humidifying Health Cigarette (HHC) of claim 1, wherein the misting mechanism provides a controlled vapor output mimicking the sensation of smoking.

4. A method for nicotine weaning, referred to as The Spina Technique (TST), comprising.
Gradually reducing nicotine doses over a specified period.
Administering higher nicotine doses in the morning to address early-day cravings.
Providing an option for PRN (as-needed) nicotine dosing to manage breakthrough cravings triggered by stress or environmental factors.

5. The method of claim 4, wherein the weaning process utilizes a Humidifying Health Cigarette (HHC) device as defined in claim 1, to provide water vapor inhalation in combination with nicotine replacement.

6. The method of claim 4, further comprising the use of a nicotine replacement system that is external to the Humidifying Health Cigarette (HHC), allowing flexibility for the user to select their preferred nicotine delivery method.

7. A system for smoking cessation, comprising.
A Humidifying Health Cigarette (HHC) device as defined in claim 1.
A nicotine replacement system for use with the HHC device, incorporating personalized weaning strategies as part of the Spina Technique (TST).
